# EUROPEAN PATENT APPLICATION

(11) **EP 4 702 976 A1**
(43) Date of publication of application: **04.03.2026**
(21) Application number: 24796873.8
(22) Date of filing: 16.04.2024
(51) Int. Cl.: A61K 31/337, A61K 31/455, A61K 31/502, A61K 38/07, A61P 21/00

(54) **AGENT FOR PREVENTING AND/OR TREATING AMYOTROPHIC LATERAL SCLEROSIS**

(30) Priority: 28.04.2023 JP 2023074457
(71) Applicant: Aichi Medical University, Aichi 480-1195 (JP); The University of Osaka, Osaka 565-0871 (JP)
(72) Inventor: SOBUE Gen, Nagakute-shi, Aichi 480-1195 (JP); TOHNAI Genki, Nagakute-shi, Aichi 480-1195 (JP); ATSUTA Naoki, Nagakute-shi, Aichi 480-1195 (JP); NAKAMURA Ryoichi, Nagakute-shi, Aichi 480-1195 (JP); OKADA Yohei, Nagakute-shi, Aichi 480-1195 (JP); OKADA Yukinori, Suita-shi, Osaka 5650871 (JP); NAMBA Shinichi, Suita-shi, Osaka 5650871 (JP)
(74) Representative: Algemeen Octrooi- en Merkenbureau B.V.
(86) International application number: PCT/JP2024/015201
(87) International publication number: WO 2024/225120

(57) **Abstract**

An object of the present invention is to provide a prophylactic and/or therapeutic agent for amyotrophic lateral sclerosis.

A prophylactic and/or therapeutic agent for amyotrophic lateral sclerosis, comprising at least one compound selected from the group consisting of paclitaxel, docetaxel, carfilzomib, olaparib, and nicotinamide.

## Description

### TECHNICAL FIELD

The present invention relates to a prophylactic and/or therapeutic agent for amyotrophic lateral sclerosis (hereinafter, may be referred to as "ALS").

### BACKGROUND ART

ALS is a progressive neurodegenerative disease that causes severe muscle atrophy and muscle weakness, and is a type of motor neuron disease. In ALS, lesions are found in a motor neuron specific manner, and as about half of the patients die or get on a respiratory system in 3 to 5 years after onset, the progress of the disease is extremely severe. The number of ALS patients in the world is estimated to be 400,000, the number of patients in Japan is about 10,000, and 1,000 to 2,000 people are newly diagnosed every year. The prevalence in Japan is estimated to be 9.9/100,000, and despite a non-rare disease, the effective therapeutic method is not currently established.

There are two types of ALS: familial ALS and sporadic ALS. Familial ALS accounts for 5 to 10% of cases, with the majority being sporadic. Although new genes have been successively discovered in recent years for familial ALS caused by mutation of a single gene, elucidation of the pathology and cause of sporadic ALS has been delayed (see, for example, Non Patent Literature 1). Among sporadic ALS, mutations in genes found in familial ALS can be found, but many cases of sporadic ALS are thought to involve multiple factors, the cause of which has not been determined.

There are many points that are not well known about the onset mechanism of ALS, and many clinical trials have been attempted, but unfortunately, there is still no effective therapeutic method for ALS. The survival period of ALS patients is said to be 3 to 5 years on average after the onset, but it has been reported that the survival period varies greatly among patients, and the survival period varies from patients requiring a ventilator in about half a year to patients who can walk without requiring a ventilator even 10 years after the onset (see, for example, Non Patent Literature 2). As described above, the diversity of the progression of ALS disease makes it difficult to analyze the results of clinical trials of ALS, and is a cause that an effective therapeutic method cannot be found.

Recently, a genome wide association study (hereinafter, may be referred to as "GWAS") using a single nucleotide polymorphism (hereinafter, may be described as "SNP") have been attempted and genetic studies involved in sporadic ALS have been conducted (see, for example, Non Patent Literature 3.). It is considered that it is possible to efficiently search for more effective therapeutic drug candidates by performing genome drug discovery from the genome wide association study of a plurality of race groups, but a specific methodology on how to proceed with genome drug discovery has not been established, and in particular, there are few examples in which genome drug discovery for a plurality of race groups has been performed.

### CITATIONS LIST

### NON-PATENT LITERATURE

Non Patent Literature 1: Finsters, J. & Burgunder, J. M., 2014, Eur. J. Med. Genet., Vol. 57 (2-3), pp.103-112
Non Patent Literature 2: Chio A. et al., 2009, Amyotroph. Lateral Scler., Vol. 10 (5-6), pp.310-23
Non Patent Literature 3: Renton, A. E., et al., 2014, Nature Neuroscience, Vol. 17 (1), pp.17-23

### SUMMARY OF INVENTION

### TECHNICAL PROBLEMS

An object of the present invention is to provide a prophylactic and/or therapeutic agent for ALS. Further, it is an object of the present invention to provide an ALS prophylactic and/or therapeutic agent that is safe for administration to humans and can suppress medical costs.

### SOLUTIONS TO PROBLEMS

As a result of intensive studies to solve the above problems, the present inventors have found an ALS risk genotype by the genome wide association study, have extracted a compound having high efficacy for ALS by overlap enrichment analysis, and have found that such a compound exhibits an effect on amelioration of symptoms of ALS, thereby completing the present invention.

That is, the present invention has been made to solve the above-described problems, and an embodiment of the present invention may include the following configurations.
(1) A prophylactic and/or therapeutic agent for amyotrophic lateral sclerosis, including at least one compound selected from the group consisting of paclitaxel, docetaxel, carfilzomib, olaparib, and nicotinamide.
(2) The prophylactic and/or therapeutic agent for amyotrophic lateral sclerosis according to (1), wherein a neurite length of a motor neuron to which the prophylactic and/or therapeutic agent for amyotrophic lateral sclerosis according to (1) is added has a higher value than that of a control.
(3) A pharmaceutical composition including a prophylactic and/or therapeutic agent for amyotrophic lateral sclerosis according to (1) or (2).
(4) A method for treating and/or preventing amyotrophic lateral sclerosis (ALS), including a step of administering an effective amount of at least one compound selected from the group consisting of paclitaxel, docetaxel, carfilzomib, olaparib, and nicotinamide to an individual in need of treatment and/or prevention of amyotrophic lateral sclerosis (ALS).
(5) At least one compound selected from the group consisting of paclitaxel, docetaxel, carfilzomib, olaparib, and nicotinamide for use in treatment and/or prevention of amyotrophic lateral sclerosis (ALS).
(6) Use of at least one compound selected from the group consisting of paclitaxel, docetaxel, carfilzomib, olaparib, and nicotinamide for producing a medicament for treatment and/or prevention of amyotrophic lateral sclerosis (ALS).

### ADVANTAGEOUS EFFECTS OF INVENTION

The present invention can provide a prophylactic and/or therapeutic agent for ALS. In addition, the prophylactic and/or therapeutic agent of the present invention is an existing drug, and thus safety for humans has already been established. In addition, both are existing drugs, and thus a synthesis method has also been established, and the drug can be delivered to a patient in need more quickly. Further, both the prophylactic and/or the therapeutic agent of the present invention are low molecular compounds, and thus the production cost can be kept low unlike biopharmaceuticals, and the medical cost burden on patients is also small.

As described above, the prophylactic and/or therapeutic agent of the present invention is effective for preventing and treating ALS, and also has an effect of being safe for administration to humans and capable of suppressing medical costs.

### BRIEF DESCRIPTION OF DRAWINGS

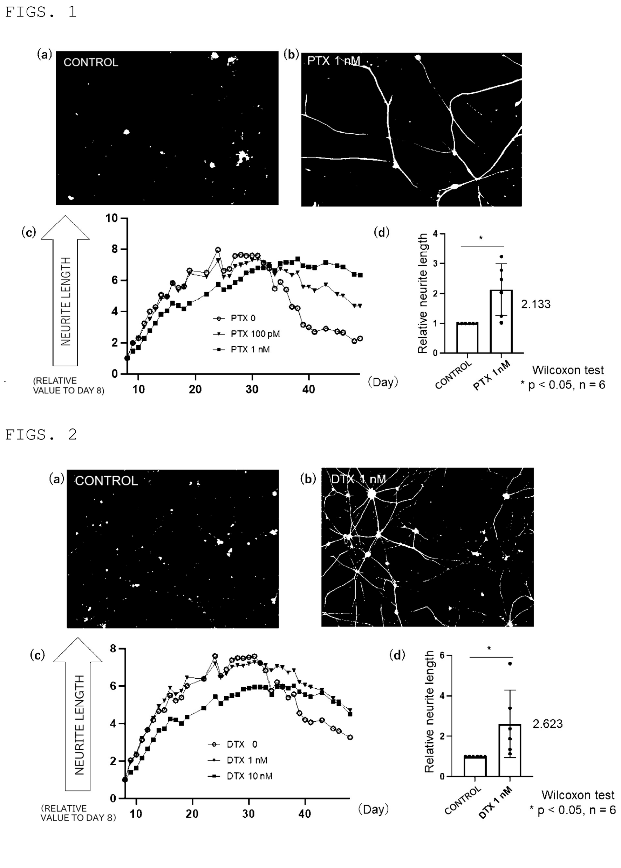

Fig. 1 is a view showing a temporal change in neurite length of a motor neuron by addition of paclitaxel. (a) and (b) are photographs of motor neurons taken on Day 38 after the start of motor neuron culture ((a) control, (b) paclitaxel 1 nM addition), (c) is a graph showing a temporal change in neurite length of a motor neuron at each concentration, and (d) is a graph showing a ratio of the motor neuron neurite length in a paclitaxel 1 nM administration group to a control on Day 42 after the start of motor neuron culture.
Fig. 2 is a view showing a temporal change in neurite length of a motor neuron by addition of docetaxel. (a) and (b) are photographs of motor neurons taken on Day 38 after the start of motor neuron culture ((a) control, (b) docetaxel 1 nM addition), (c) is a graph showing a temporal change in neurite length of a motor neuron at each concentration, and (d) is a graph showing a ratio of the motor neuron neurite length in a docetaxel 1 nM administration group to a control on Day 42 after the start of motor neuron culture.

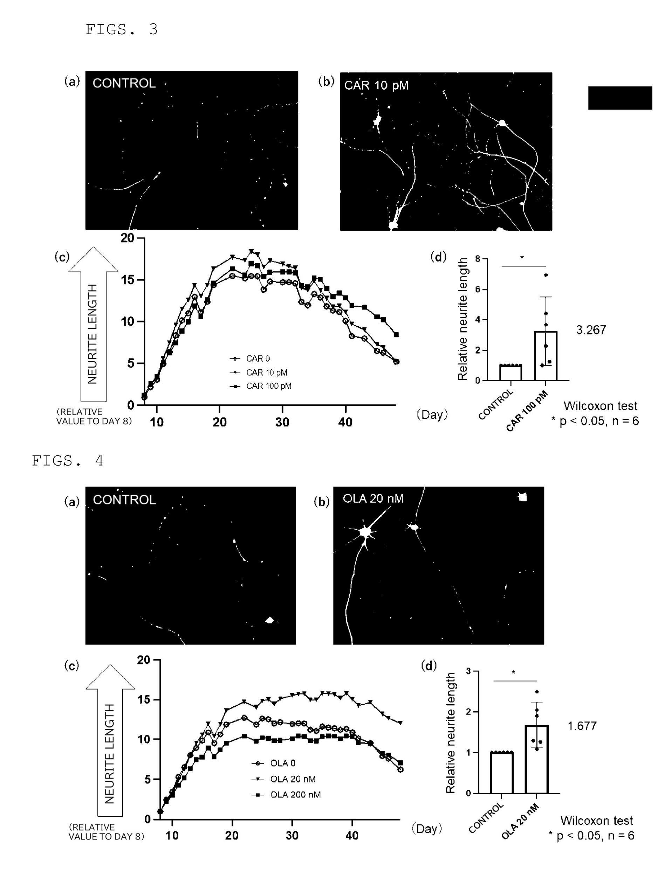

Fig. 3 is a view showing a temporal change in neurite length of a motor neuron by addition of carfilzomib. (a) and (b) are photographs of motor neurons taken on Day 30 after the start of motor neuron culture ((a) control, (b) carfilzomib 10 pM addition), (c) is a graph showing a temporal change in neurite length of a motor neuron at each concentration, and (d) is a graph showing a ratio of the motor neuron neurite length in a carfilzomib 100 pM administration group to a control on Day 42 after the start of motor neuron culture.
Fig. 4 is a view showing a temporal change in neurite length of a motor neuron by addition of olaparib. (a) and (b) are photographs of motor neurons taken on Day 30 after the start of motor neuron culture ((a) control, (b) olaparib 20 nM addition), (c) is a graph showing a temporal change in neurite length of a motor neuron at each concentration, and (d) is a graph showing a ratio of the motor neuron neurite length in an olaparib 20 nM administration group to a control on Day 42 after the start of motor neuron culture.

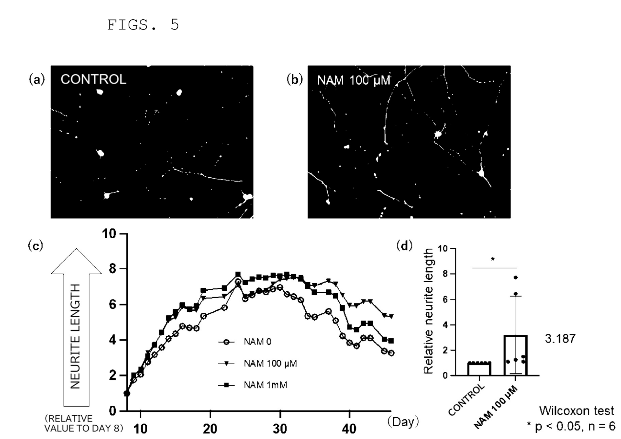

Fig. 5 is a view showing a temporal change in neurite length of a motor neuron by addition of nicotinamide. (a) and (b) are photographs of motor neurons taken on Day 38 after the start of motor neuron culture ((a) control, (b) nicotinamide 100 µM addition), (c) is a graph showing a temporal change in neurite length of a motor neuron at each concentration, and (d) is a graph showing a ratio of the motor neuron neurite length in a nicotinamide 100 µM administration group to a control on Day 42 after the start of motor neuron culture.

### DESCRIPTION OF EMBODIMENT

The ALS prophylactic and/or therapeutic agent of the present invention is based on the finding of an unexpected new finding that when a compound extracted by the genome wide association study and overlap enrichment analysis is added to iPS cells derived from ALS patients, the neurite length of motor neurons is elongated.

### <Detection of ALS risk gene polymorphism by genome wide association study>

The method for detecting ALS risk gene polymorphism from the genome wide association study is not particularly limited, and reference can be made to Nakamura R. et al. Commun Biol. 2020 Sep 23; 3 (1): 526. In the literature, gene-based analysis of all multi-ethnic datasets has been used to discover genes significantly associated with ALS. As a method for detecting SNP, a known method can be used.

### <Extraction of candidate compound>

The drug discovery method using the obtained risk gene polymorphism data is not particularly limited, but the compound can be searched for by a method such as enrichment analysis of a disease risk gene (technique for searching for a drug targeting a disease risk gene based on a drug category to which the disease risk gene belongs) or correlation analysis of gene expression level control (technique for estimating gene expression level control by disease genome and searching for a compound for controlling gene expression level in a reverse direction).

When the enrichment analysis of the disease risk gene is performed, a method of extracting a gene group to be noted for the enrichment analysis from among the expression-fluctuating genes is not limited, and examples thereof include a method of extracting based on an amount of change in gene expression, and a method of extracting based on network analysis such as coexpression network analysis or interaction analysis between proteins. When correlation analysis of gene expression level control is performed, reference can be made to Konuma T. et al., Human Molecular Genetics, 2021, Vol. 30, No. 3-4.

In the present invention, candidate compounds have been searched using enrichment analysis of disease risk genes. Specifically, the risk gene polymorphism data and the gene polymorphism-gene expression-compound database have been integrated, and a highly effective compound category has been extracted by overlap enrichment analysis, and it has been found that such a compound has an ALS therapeutic effect, thereby completing the present invention.

### <ALS prophylactic and/or therapeutic agent>

The ALS prophylactic and/or therapeutic agent of the present invention includes at least one compound selected from the group consisting of paclitaxel, docetaxel, carfilzomib, olaparib, and nicotinamide. Two or more types thereof may be combined. In addition, the five compounds also include a pharmaceutically acceptable salt, solvate, or prodrug.

The salt is not particularly limited as long as it is a pharmaceutically acceptable salt, and examples thereof include inorganic acid salts such as a hydrochloride, a sulfate, a hydrobromide, a nitrate, and a phosphate; organic acid salts such as an acetate, a mesylate, a succinate, a maleate, a fumarate, a citrate, and a tartrate; alkali metal salts such as a sodium salt and a potassium salt; alkaline earth metal salts such as a magnesium salt and a calcium salt; metal salts such as an aluminum salt and a zinc salt; ammonium salts such as an ammonium salt and a tetramethylammonium salt; organic amine addition salts such as morpholine and piperidine; amino acid addition salts such as glycine, phenylalanine, lysine, aspartic acid, and glutamic acid.

The solvate is not particularly limited as long as it is a pharmaceutically acceptable solvate, and examples thereof include a hydrate and an organic solvate.

As the five compounds, known substances can be used. The five compounds can be produced by known methods, and commercially available compounds can be obtained and used. It is preferable to use those conforming to the Japanese Pharmacopoeia. Synthesis is possible by organic synthesis, and thus the production cost can be kept low unlike biopharmaceuticals.

Paclitaxel and docetaxel are known as taxane-based anticancer agents. Examples of paclitaxel include TAXOL (registered trademark) and an albumin-engineered nanoparticle formulation of paclitaxel (ABRAXANE (registered trademark)), and examples of docetaxel include TAXOTERE (registered trademark). Carfilzomib is known as a therapeutic drug for multiple myeloma and examples thereof include Kyprolis (registered trademark). Olaparib is used for the treatment of ovarian cancer, breast cancer, prostate cancer, pancreatic cancer, and the like, and examples thereof include Lynparza (registered trademark). Nicotinamide may be blended in cosmetics for dermatitis such as acne and eczema, stomatitis, and cosmetic purposes. However, none of the five compounds has been applied to ALS. It has been surprising that the above five compounds have an effect on ALS as in the present invention because the previously known indication development mechanism of the above five compounds and ALS development mechanism are different.

### <Administration method and dose>

As the subject to be administered with the prophylactic and/or therapeutic agent of the present invention, it is preferable to perform administration to a patient in whom treatment of ALS is desired. The prophylactic and/or therapeutic agent of the present invention can also be used as the prophylactic agent for onset of ALS for a subject suspected of having fear of onset of ALS in the future based on a family history or other clinical test data although the subject has no symptom of ALS, other than a patient diagnosed with ALS. The degree of the symptom may be any of an initial stage, an intermediate stage, and an end stage.

In addition, the scope of application of the present invention is not limited to Japanese. That is, the present invention is also applicable to non-Japanese Mongoloid (such as Chinese) and other ethnic groups (such as Caucasoid) .

The dosage form of the prophylactic and/or therapeutic agent of the present invention is not particularly limited, and the prophylactic and/or therapeutic agent can be formulated as an oral preparation or a parenteral preparation such as a tablet, a powder, a granule, a capsule, a solution, an emulsion, an elixir, a suspension, a syrup, a troche, an inhalant, a suppository, an injection, an ointment, an eye ointment, an eye drop, a nasal drop, an ear drop, a cataplasm, or a lotion.

The present invention may be a pharmaceutical composition containing the prophylactic and/or therapeutic agent. That is, the pharmaceutical composition of the present invention contains the prophylactic and/or therapeutic agent as an active ingredient, and may contain a pharmaceutically acceptable excipient, for example, a solid, semi-solid, or liquid diluent, a dispersant, a filler, and a carrier as necessary. Further, as long as the effect of the present invention is not impaired, a stabilizer, a preservative, a pH adjuster, a binder, a disintegrant, a surfactant, a lubricant, a flowability accelerator, a flavoring agent, a coloring agent, a perfume preservative, a medium, a physiological saline, or the like may be included as an additive.

An appropriate dose per day of the prophylactic and/or therapeutic agent of the present invention depends on the type, activity, administration route and administration target (patient's age, sex, presence or absence of previous disease, and the like) of the compound contained in the prophylactic and/or therapeutic agent, presence or absence of a concomitant drug, and other factors. Therefore, in order to obtain an optimal therapeutic effect, it is preferable that a medical professional adjusts the dose or changes the administration route as necessary. The dose of the prophylactic and/or therapeutic agent of the present invention is preferably 10 ng/Kg body weight to 100 mg/kg body weight per day.

The upper limit of the amount of paclitaxel is preferably 5 mg/Kg body weight, and more preferably 2 mg/Kg body weight. The upper limit of the amount of the docetaxel is preferably 5 mg/Kg body weight, and more preferably 1.5 mg/Kg body weight. The upper limit of the amount of carfilzomib is preferably 1 mg/Kg of body weight, and more preferably 0.5 mg/Kg of body weight. The upper limit of the amount of olaparib is preferably 10 mg/Kg body weight, and more preferably 5 mg/Kg body weight. The upper limit of the amount of nicotinamide is preferably 6 mg/Kg body weight, and more preferably 3 mg/Kg body weight. The number of administrations is not particularly limited as long as the above-described dose is satisfied. The drug may be administered once a day or approximately 2 to 4 times a day.

### <Neurite outgrowth of motor neurons>

In the prophylactic and/or therapeutic agent of the present invention, it is preferable that the neurite outgrowth of motor neurons can be confirmed by the following method.

ALS is characterized by loss of motor neurons in the primary motor area, brainstem, and spinal cord. The loss of motor neurons disrupts basic basal movements such as respiration. ALS is known to decrease the length of the neurite of the motor neuron, and thus in the present specification, the degree of neurite outgrowth of motor neurons due to the addition of the compound is confirmed.

In the present invention, the neurite length of the motor neuron preferably has a higher value than that of the control. The higher value means that the neurite length of the motor neuron in the group to which the compound of the present invention is added is longer than the neurite length of the motor neuron in the group to which the compound of the present invention is not added (control). The neurite length is preferably measured through the following steps.
(a) A step of inducing differentiation of motor neurons from iPS cells derived from a sporadic ALS patient.
(b) A step of bringing the motor neurons induced to differentiate in the step (a) into contact with the compound of the present invention.
(c) A step of culturing the motor neurons that have been brought into contact with the compound of the present invention and motor neurons that have not been brought into contact with the compound of the present invention (referred to as "control").
(d) A step of measuring neurite lengths of the motor neurons obtained in the step (c).

In the present invention, it is preferable that the neurite length of the motor neuron brought into contact with the compound of the present invention, which is measured in the step (d), has a higher value than that of the control.

Each step will be described in detail. In the step (a), iPS cells derived from the sporadic ALS patient are differentiated into motor neurons. The method is not particularly limited, and it is preferable to use a bacterial dish method in which iPS cells are adhered to a culture dish subjected to coating treatment and cultured in an arbitrary medium with appropriately changing additives.

In the step (b), the differentiation-induced motor neurons are brought into contact with the compound of the present invention. In the present invention, the day on which iPS cells are induced to differentiate into motor neurons after culturing for about 14 days by the bacterial dish method is defined as the "day of starting motor neuron culture". The culture conditions are preferably 37°C, 5% CO₂, and 5% O₂.

The contact with the compound of the present invention is preferably performed on Day 6 to Day 10, preferably Day 8, and more preferably Day 9 after the start of the motor neuron culture. The contact method is not particularly limited, and it is preferable to add the compound of the present invention to the differentiation-induced motor neurons.

The culture in the step (c) is preferably performed under conditions of 37°C, 5% CO₂, and 5% O₂.

The measurement of the neurite length in the step (d) can be performed using methods well known to those skilled in the art. For example, motor neurons may be fluorescently labeled and measured using a cell image analyzer (in-cell analyzer).

It is preferable that the neurite length of the motor neuron to which the compound of the present invention is added is longer than that of the control. In this case, it can be determined that the compound of the present invention is effective as an ALS therapeutic drug. The neurite length of the motor neuron to which the compound of the present invention is added preferably has a higher value than that of the control on Days 30 to 50 after the day of starting the motor neuron culture, and more preferably has a higher value on Days 35 to 45.

On Day 42 after the start of the motor neuron culture, the neurite length of the motor neuron to which the compound of the present invention is added is preferably 1.05 times or more, and more preferably 1.10 times or more, as compared with that of the control.

### [Examples]

The present invention will be specifically described with reference to the following examples, but the present invention is not limited thereto.

### <Differentiation of motor neurons using iPS cells derived from sporadic ALS patient and confirmation of drug efficacy>

The human lymphoblastoid cell line (LCL) was immortalized by infecting peripheral blood mononuclear cells obtained from sporadic ALS patients registered in JaCALS with EB virus according to the protocol of SRL, and established as a cell line. Each plasmid expressing six factors (OCT4, SOX2, KLF4, l-MYC, LIN28, p53) was introduced into the LCL by an electroporation method using Neon Transfection System to prepare iPS cells.

The prepared iPS cells were coated with 0.1% gelatin and plated on a dish in which SNL mouse fibroblast feeder cells treated with mitomycin C were cultured. The next day, the medium was changed to an hES medium (refer to Table 1). As the established iPS cell lines, eight lines SALS-001 to SALS-008 were used.

**[Table 1]**

| hES medium |
|---|
| • DMEM/F12 medium |
| • 20% KSR |
| • 2 mM L-glutamine |
| • 1% non-essential amino acid (NEAA) |
| • 0.1 mM 2-metcaptoethanol (2ME) |
| • 4 ng/mL basic FGF |

Differentiation induction into motor neurons was performed using a bacterial dish method. Specifically, the iPS cells cultured on feeder cells were removed with a stripping solution and transferred to a bacterial dish, and cultured in an hES medium. The next day, culture medium was replaced with an EB medium, and 300 nM LDN-193189, 3 µM SB431542, and 3 µM CHIR99021 were added thereto. Further, the next day, the culture medium was replaced with an EB medium (refer to Table 2) to which LDN-193189, SB431542, CHIR99021, and 3 µM retinoic acid were added. After 2 days, 5 days, and 8 days, the culture solution was further replaced with an EB medium to which retinoic acid and 1 µM purmorphamine were added. The iPS cells were harvested 4 days after the last medium exchange. These were seeded in a 6-well plate and cultured in an MN medium (refer to Table 3). This induced differentiation into motor neurons.

**[Table 2]**

| EB medium |
|---|
| • DMEM/F12 medium |
| • 5% KSR |
| • 2 mM L-glutamine |
| • 1% NEAA |
| • 0.1 mM 2ME |

**[Table 3]**

| MN medium | |
|---|---|
| • KBM medium | • 10 ng/mL GDNF |
| • 2% B27 | • 50 nM retinoic |
| • 1% NEAA | acid |
| • 10 µM cAMP | • 500 nM purmorphamine |
| • 10 ng/mL BDNF | • 200 ng/mL ascorbic acid |
| | • DAPT (100 µM) |

On Day 3 after the start of the motor neuron culture, the medium was replaced with opti-mem (Thermo Fisher Scientific Inc.), and lentivirus (HB9e438 Venus) was added to infect the gene. Motor neurons were labeled with fluorescent proteins. After 6 hours, the medium was replaced with an MN medium. On Day 7 after the start of the motor neuron culture, the motor neurons were reseeded in a 96-well plate. On Day 9 after the start of the motor neuron culture, as shown in Table 4, the compounds in the same table were dissolved in a solvent and administered at each concentration, and incubated in a multi-gas incubator under the conditions of 37°C, 5% CO₂, and 5% O₂.

**[Table 4]**

| Chemical name | Dose concentration | Solvent |
|---|---|---|
| Paclitaxel (FUJIFILM Wako Pure Chemical Corporation) | 10 pM, 100 pM, 1 nM | DMSO |
| Docetaxel (FUJIFILM Wako Pure Chemical Corporation) | 100 pM, 1 nM, 10 nM | DMSO |
| Carfilzomib (AdipoGen Life Sciences, Inc.) | 10 pM, 100 pM, 1 nM, 10 nM | DMSO |
| Olaparib (Chemscene LLC) | 20 nM, 200 nM, 2 µM, 20 µM | DMSO |
| Nicotinamide (FUJIFILM Wako Pure Chemical Corporation) | 100 µM, 1 mM, 10 mM | Physiological saline |

### <Analysis of neurite length>

For the motor neurons induced to differentiate as described above, the temporal change in neurite length after administration of the compound was measured. For the measurement, IN Cell Analyzer 6000 (GE Healthcare Technologies Inc.) was used. The results are shown in Figs. 1 to 5. (a) and (b) in each drawing are images on Day 30 or Day 38 after the start of the motor neuron culture. It can be seen that the neurite length of the motor neuron is longer than that of the control in any compound.

In addition, (c) in each drawing is a graph illustrating a temporal change in neurite length. The vertical axis represents the neurite length (relative value) with respect to Day 8 after the start of the motor neuron culture, and the horizontal axis represents the number of culture days from the day of starting the motor neuron culture. It can be seen that the neurite length of the motor neuron to which the compound of the present invention is added is longer than the neurite length of the control motor neuron.

(d) in each drawing represents the ratio of the neurite length of the motor neuron in the compound administration group (n = 6) to the control on Day 42 after the start of the motor neuron culture. P values were calculated using the Wilcoxon test. It can be seen that the neurite length of the motor neuron to which the compound is added is 1.05 times or more longer than that of the control.

As is apparent from these results, it has been observed that the neurite length of the motor neuron was prolonged by adding the compound of the present invention. It has been revealed that the compound of the present invention is effective for prevention and treatment of ALS.

### INDUSTRIAL APPLICABILITY

The present invention can provide a prophylactic and/or therapeutic agent for amyotrophic lateral sclerosis. The prophylactic and/or therapeutic agent of the present invention is an existing drug, and thus safety and synthetic methods for humans have already been established. Therefore, the prophylactic and/or therapeutic agent of the present invention is effective for preventing and treating ALS, and is safe for administration to humans and medical costs can be reduced.

## Claims

1. A prophylactic and/or therapeutic agent for amyotrophic lateral sclerosis, comprising at least one compound selected from the group consisting of paclitaxel, docetaxel, carfilzomib, olaparib, and nicotinamide.

2. The prophylactic and/or therapeutic agent for amyotrophic lateral sclerosis according to claim 1, wherein a neurite length of a motor neuron to which the prophylactic and/or therapeutic agent for amyotrophic lateral sclerosis according to claim 1 is added has a higher value than that of a control.

3. A pharmaceutical composition comprising a prophylactic and/or therapeutic agent for amyotrophic lateral sclerosis according to claim 1 or 2.
